Europäisches Patentamt

European Patent Office

Office européen des brevets

(19)

(11) Publication number : **0 507 586 A2**

# EUROPEAN PATENT APPLICATION

(12)

(21) Application number : 92302912.8

(22) Date of filing : 02.04.92

(51) Int. Cl.$^5$ : **G01N 33/68,** G01N 33/576,
// G01N33/543

(30) Priority : 03.04.91 US 679270

(43) Date of publication of application :
07.10.92 Bulletin 92/41

(84) Designated Contracting States :
AT BE CH DE DK ES FR GB GR IT LI LU NL PT
SE

(71) Applicant : SYNTEX (U.S.A.) INC.
3401 Hillview Avenue
Palo Alto California 94304 (US)

(72) Inventor : Rejman, John J.
13619 Highway 70 East
Lenior City, TN 37771 (US)
Inventor : Weng, Litai
995 N. california Avenue
Palo Alto, CA 94303 (US)
Inventor : Choo, Sae H.
20043 Merritt Drive
Cupertino, CA 95014 (US)

(74) Representative : Nicholls, Kathryn Margaret et
al
Mewburn Ellis, 2 Cursitor Street
London EC4A 1BQ (GB)

(54) Immunoassay for immunoglobulins.

(57)    A method for carrying out an immunoassay for an immunoglobulin in which a sample suspected of containing the immunoglobulin and reagents useful for detecting the immunoglobulin of interest are combined in a single step in an aqueous medium, wherein one of the reagents includes a small molecule bound to a first antigen capable of binding to the immunoglobulin and another includes a signal generating means bound to a second antigen capable of binding to the immunoglobulin.

EP 0 507 586 A2

Immunoglobulins are a class of proteins which function as antibodies and are produced by specialized cells (β-lymphocytes) of an immuno-competent organism (i.e., an organism with an intact immune system) in response to the presence of a molecule which is foreign to the organism.

There are five categories of immunoglobulins (Ig) based upon their chemical structures and the different functions they perform in the immune system: IgA, IgD, IgE, IgG and IgM. Because their presence is usually an indication of infection, immunoassays to identify the presence of immunoglobulins are useful for the early diagnosis of infection.

Hepatitis is an infectious disease for which an immunoassay based upon immunoglobulin detection is useful. Such an assay finds usefulness not only as a purely diagnostic tool, but also in pre-transfusion screening of blood and in screening potential blood donors. Hepatitis B or serum hepatitis occurs worldwide and is commonly seen among drug users who transmit the disease via shared hypodermic needles. Hepatitis B is usually diagnosed by confirmation of the presence of viral antigens. Assay kits for the hepatitis B surface antigen, the core antigen and antibodies directed against these antigens are commercially available. Assays for the surface antigen (HBsAg) are preferred since HBsAg is detectable in the serum before symptoms appear and up to several months after onset of the disease. HBsAg is the first virus marker to appear.

U. S. Patent No. 4,292,403 pertains to an Ig immunoassay where bound anti-Ig is incubated with a sample, then incubated with an antigen capable of binding to Ig, and lastly incubated with a labeled anti-antigen fragment. Each incubation is followed by a wash step and the antibody is bound by coupling to a solid carrier by means of covalent bonds or by adsorption.

U. S. Patent No. 4,273,756 describes an Ig assay where bound anti-Ig is contacted with a test sample, washed, then reacted with a labeled antigen capable of binding to Ig.

U. S. Patent No. 4,020,151 pertains to an Ig assay where Ig in a sample is first sorbed directly onto a nonimmunological surface, followed by reaction with labeled anti-Ig.

U. S. Patent No. 4,837,167 describes a simultaneous sandwich assay to determine multi-determinant antigens using high affinity monoclonal anti-Ig.

Bradley, et al., Journal of Clinical Microbiology 5(5):521-530 (1977) describes an assay for IgM class immunoglobulins related to the HAV virus where HAV and radiolabeled IgG anti-HAV is added to a test tube coated with patient serum suspected of containing IgM.

U. S. Patent No. 4,474,878 pertains to a sandwich enzyme immunoassay for hepatitis antigen where a sample is incubated with bound antibody, then incubated with enzyme labeled antibody.

U. S. Patent No. 4,818,688 describes an assay for antibodies to hepatitis B core antigen (HBcAg) where labeled antibody competes with antibody in the sample for binding to immobilized HBcAg.

U. S. Patent No. 4,098,876 pertains to a reverse sandwich assay for hepatitis associated antigen where a sample is incubated with labeled antibody followed by incubation with immobilized antibody.

Bussian, et al., Clinical Chemistry 34(6):1315 (1988) describes an assay for IgM antibodies to HAV: sample is incubated with biotinylated anti-IgM then with a streptavidin coated solid phase; the complex is washed then mixed with HAV; and the new complex is washed and then mixed with labeled anti-HAV.

U. S. Patent No. 4,271,140 pertains to the use of biotin and avidin in a double receptor complex where biotin and avidin form a nonimmune, reversible binding system.

U. S. Patent No. 4,298,685 describes the use of a biotinylated antibody bound to an avidin coated solid phase, where analyte present in a sample competes with unlabeled analyte for binding to the antibody.

U. S. Patent No. 4,935,339 pertains to a one step immunoassay where a biotinylated first immunological binding partner, a test sample, a labeled second immunological binding partner and a biotin-binding protein bound to a carrier are all combined.

U. S. Patent No. 4,343,896 describes an assay using two antibodies from different animal species which are raised against the same antigen, where one antibody is labeled and the other antibody is insolubilized by a third antibody.

U. S. Patent Application Serial No. 07/389,659 (corresponding to European Patent Publication No. 411,945, Japanese Patent Application No. 206628/90 and Canadian Patent Application SN 2,022,517-3) pertains to an immunoassay using a small molecule bound to a specific binding pair member and a second small molecule bound to a second specific binding pair member, where both binding pair members are capable of binding the analyte. The assay also uses a receptor for the first small molecule, bound to a support and a receptor for the second small molecule bound to a label.

According to a first aspect of the invention, a method for carrying out a qualitative immunoassay for specific immunoglobulins is provided wherein the following components are combined without prior incubation in an aqueous medium and incubated: a sample suspected of containing the immunoglobulin of interest, a small molecule bound to a first antigen capable of binding to the immunoglobulin (antigen-small molecule conjugate), a signal generating means bound to a second antigen capable of binding to the immunoglobulin (antigen-signal generating means conjugate), and a support to which is bound a receptor for the small molecule. The first and second antigens can be the same antigen, the only difference being that some antigen will be bound to the small molecule in the antigen-small molecule conjugate and some antigen will be bound to the signal generating means in the antigen-signal generating means conjugate. The medium is then separated from the support. The medium or the support is observed for the presence or amount of the signal generating means, the presence or amount thereof being related to the presence or amount of immunoglobulin in the sample.

Another embodiment of the invention describes a method for carrying out an immunoassay for an IgG-type immunoglobulin wherein the following components are combined in an aqueous medium and incubated: a sample suspected of containing IgG, a biotinylated first antigen capable of binding to IgG, a signal generating means bound to a second antigen capable of binding to IgG, and a receptor for biotin bound to a support. The medium is then separated from the support. The medium or the support is observed for the presence or amount of the signal generating means, the presence or amount thereof being related to the presence or amount of IgG in the sample.

Yet another embodiment of the invention describes an assay for detecting an IgG-type immunoglobulin suspected of being present in a sample wherein a complex is formed in direct relation to the amount of IgG in the sample, the complex being:

$$\text{Label-}Ag_2: \text{IgG}: Ag_1\text{-}X:Y\text{-support}$$

where $Ag_1$-X is a small molecule (X) bound to a first antigen ($Ag_1$) capable of binding to IgG, Label-$Ag_2$ is a label bound to a second antigen ($Ag_2$) capable of binding to IgG, and Y-support is a receptor for the small molecule bound to a support.

Another embodiment of the invention describes a composition of matter consisting of a conjugate of a biotinylated antigen bound to an immunoglobulin and a labeled antigen bound to the immunoglobulin.

Still another embodiment of the invention describes a composition of matter:

$$\text{Label-}Ag_2: \text{IgG}: Ag_1\text{-}X:Y\text{-support}$$

where IgG is an IgG-type immunoglobulin, $Ag_1$-X is a small molecule (X) bound to a first antigen ($Ag_1$) capable of binding to IgG, Label-$Ag_2$ is a label bound to a second antigen ($Ag_2$) capable of binding to IgG, and Y-support is a receptor for the small molecule bound to a support.

In another embodiment of the invention a kit for carrying out an immunoassay for an immunoglobulin is described which comprises in packaged form: a conjugate of a small molecule and a first antigen capable of binding the immunoglobulin, a conjugate of a label and a second antigen capable of binding to the immunoglobulin, and a support comprised of a surface bound to a receptor for the small molecule.

The present invention provides a one step simultaneous immunoassay for specific immunoglobulins (Ig), preferably IgG class and in particular IgG for hepatitis B surface antigen (HBsAg). A complex is formed between the Ig analyte, an antigen-small molecule conjugate and an antigen-signal generating means conjugate. The small molecule can allow for the complex to be insolubilized by means of a receptor for the small molecule bound to a support. All the reagents are mixed together and then incubated. This single incubation is followed by a wash step, which is then followed by the addition of any remaining members of the signal generating means.

There are several advantages to the present invention. One advantage is the elimination of the numerous incubation and wash steps commonly seen in state of the art techniques. Elimination of several of these steps provides for an assay format that is less labor intensive. In some instances, this invention may use a slightly higher amount of reagents, but this is far outweighed by the labor-saving advantage. Another advantage is that by eliminating the need for several incubation and wash steps, this invention provides for shorter assay time. Surprisingly, even with the elimination of the numerous incubation and wash steps seen in state of the art assay techniques, both an adequate signal can be obtained to enable detection of the analyte and minimal interference occurs between the first and second antigen in binding to the analyte. Before proceeding further with the description of the specific embodiments of the present invention, a number of terms will be defined.

"Immunoglobulin" means the compound to be measured in a sample that is the material of interest, i.e., the analyte. The sample is preferably serum or plasma. The Immunoglobulin (Ig) may be directed against a causative agent of a disease state. The immunoglobulin can be class specific such as IgA, IgD, IgE, IgG and IgG, with a molecular weight that will generally vary from about 160,000 to about $10^6$. As used herein the term "immunoglobulin" shall refer to the analyte and the term "antibody" shall be used in reference to the reagents.

"Antigen" means any compound capable of binding to the immunoglobulin of interest, and against which antibodies can be raised. For example, the antigen can be hepatitis B surface antigen.

"Ligand" means any organic compound for which a receptor naturally exists or can be prepared.

"Receptor" means any compound or composition capable of recognizing a particular spatial and polar organization of a molecule, e.g., epitopic or determinant site. Illustrative receptors include naturally occurring receptors, e.g., thyroxine binding globulin, antibodies, enzymes, Fab fragments, lectins, protein A, complement component C1q, avidin, streptavidin and the like. Preferably, if the small molecule is biotin, the receptor for biotin may be avidin or streptavidin.

"Support" means any non-porous or porous surface. Typical support surfaces include glass or plastic beads, latex or cellulose particles, glass or cellulose filter paper, nitrocellulose membranes, polystyrene plates, magnetic particles, plastic tubes or vessels, and the like. The support may be of any convenient material to which a receptor can be non-diffusively bound and which does not dissolve in or react adversely with the aqueous medium. Usually the support will be plastic such as polystyrene, polycarbonate, polyacrylate, polyurethane, polyvinylchloride, teflon and the like or it may be metallic such as steel, nickel, copper, gold or chromium dioxide and preferably will be ceramic including, for example, quartz, glass, and the like. When the support is a matrix of beads, the beads will usually be of a defined approximately uniform, size, preferably 0.2-2.5mm, and will have either a rough or smooth surface, preferably smooth. Preferably the beads are rounded or oblong, usually approximately spherical and have surface properties which minimize non-specific binding. As used in the immunoassays of the invention, the support will have bound to it a material such as a receptor, preferably such as an antibody, avidin, apoenzyme, repressor protein, intrinsic factor and the like.

Binding of materials to the support or surface may be accomplished by well-known techniques, commonly available in the literature. See, for example, "Immobilized Enzymes," Ichiro Chibata, Halsted Press, New York (1978) and Cuatrecasas, J. Biol. Chem., 245:3059 (1970). The surface can have any one of a number of shapes, such as strip, rod, particle, including bead, and the like.

The surface will usually be polyfunctional or be capable of being polyfunctionalized or be capable of binding a receptor, for example, through specific or non-specific covalent or non-covalent interactions. A wide variety of functional groups are available or can be incorporated. Functional groups include carboxylic acids, aldehydes, amino groups, cyano groups, ethylene groups, hydroxyl groups, mercapto groups and the like. The manner of linking a wide variety of compounds to particles is well known and is amply illustrated in the literature. See for example Cuatrecasas, J. Biol. Chem. 245:3059 (1970). The length of a linking group to the material being linked may vary widely, depending upon the nature of the material being linked, the effect of the distance between the material being linked and the particle on the hybridization of the sequences and the like. The material being linked will be substantially bound to the outer surface of the particle.

Particles employed as the surface can be fluorescent either directly or by virtue of fluorescent compounds or fluorescers bound to the particle in conventional ways. The fluorescers will usually be dissolved in or bound covalently or non-covalently to the particle and will frequently be substantially uniformly bound through the particle. Fluoresceinated latex particles are taught in U.S. Patent No. 3,853,987.

"Small molecule" means an organic or organometallic group, having a molecular weight of from 100-2000, preferably 150-1000, usually bonded to a receptor or a support and for which a receptor exists or can be prepared. Examples of small molecules useful in the invention include derivatives of biotin, lysergic acid, brucine, fluorescein, vitamin $B_{12}$ and in general molecules that are not usually found in high concentration in the samples to be assayed. For biological samples, highly toxic molecules and synthetically derived molecules other than drugs are often preferred.

"Signal generating means" means one or more components, at least one component being a label, which generate a signal that relates to the presence or amount of immunoglobulin in a sample. The signal generating means includes all of the reagents required to produce a measurable signal. The label can be isotopic or non-isotopic, usually non-isotopic, including catalysts such as an enzyme, a chromogen such as a fluorescer, dye or chemiluminescer, a metallic particle, and so forth. The label can be conjugated directly to the receptor for the antigen or the label can be conjugated to a receptor for a small molecule, where the small molecule is bound to the receptor for the antigen. Components of the signal generating means may be chemiluminescers, radioactive substances, coenzymes, substances that react with enzymic products, enzymes, and catalysts, solid particles, fluorophors, chromophors, gold particles and the like. The signal generating means provides a signal detectable by external means, preferably by measurement of the degree of aggregation of particles or by use of electromagnetic radiation, desirably by visual examination. For the most part, the signal generating means will involve, a chromophoric substrate and enzyme, where chromophoric substrates are enzymatically converted to dyes which absorb light in the ultraviolet or visible region, phosphors, fluorescers or chemiluminescers, radioactive atoms, electroactive groups, and the like.

In the methods according to the present invention, the assay medium suspected of containing an immunoglobulin may include a small molecule bound to a first antigen capable of binding to the immunoglobulin, a signal generating means bound to a second antigen capable of binding to the immunoglobulin, and a support to which is bound a receptor for said small molecule. The signal generating means is preferably a label selected from the group consisting of enzymes, fluorophors, chemiluminescers and metallic particles, and most preferably said label is an enzyme selected from the group consisting of peroxidase, β-galactosidase, urease, alkaline phosphatase and Q-beta-replicase; the signal generating means may also comprise a second small molecule bound to said second antigen and a receptor for said second small molecule bound to a label selected from the group consisting of enzymes, fluorophors, chemiluminescers and metallic particles, preferably wherein said molecule is a fluorescein derivative, said receptor is an antibody, and said label is an enzyme selected from the group consisting of peroxidase, β-galactosidase, urease, alkaline phosphatase and Q-beta-replicase.

Also, in an immunoassay method according to the present invention for an IgG-type immunoglobulin, which includes a biotinylated first antibody capable of binding said IgG, a signal generating means bound to a second antigen capable of binding to said IgG, and a receptor for biotin bound to a support, the signal generating means may be a label selected from the group consisting of enzymes, fluorophors, chemiluminescers and metallic particles, and preferably said label is an enzyme selected from the group consisting of peroxidase, β-galactosidase, urease, alkaline phosphatase and Q-beta-replicase; the signal generating means may also comprise a second small molecule bound to the second antigen and a receptor for the second small molecule bound to a label selected from the group consisting of enzymes, fluorophors, chemiluminescers and metallic particles, preferably wherein the molecule is a fluorescein derivative, the receptor is an antibody, and the label is an enzyme selected from the group consisting of peroxidase, β-galactosidase, urease, alkaline phosphatase and Q-beta-replicase.

The signal generating means can include at least one catalyst, usually an enzyme, and at least one substrate and may include two or more catalysts and a plurality of substrates, and may include a combination of enzymes, where the substrate of one enzyme is the product of the other enzyme. The operation of the signal generating means is to produce a product which provides a detectable signal related to the amount of immunoglobulin in the sample.

A large number of enzymes and coenzymes useful in a signal generating system are indicated in U.S. Patent No. 4,275,149, columns 19 to 23, U.S. Patent No. 4,318,980, columns 10 to 14, and U.S. Patent No. 4,868,104, column 7. A number of enzyme combinations are set forth in U.S. Patent No. 4,275,149, columns 23 to 28, which combinations can find use in the subject invention.

Of particular interest are enzymes which involve the production of hydrogen peroxide and the use of the hydrogen peroxide to oxidize a dye precursor to a dye. Particular combinations include saccharide oxidases, e.g., glucose and galactose oxidase, or heterocyclic oxidases, such as urease and xanthine oxidase, coupled with an enzyme which employs the hydrogen peroxide to oxidize a dye precursor, that is, a peroxidase such as horseradish peroxidase, lactoperoxidase, or microperoxidase. Additional enzyme combinations may be found in the patent references mentioned above. When a single enzyme is used as a label, other enzymes may find use such as hydrolases such as alkaline phosphatase and β-galactosidase. Alternatively, luciferases such as firefly luciferase and bacterial luciferase may be used.

Illustrative coenzymes which find use include NAD[H]; NADP[H], pyridoxal phosphate; FAD[H]; FMN[H], etc., usually coenzymes involving cycling reactions, see particularly U.S. Patent No. 4,318,980.

The product of the enzyme reaction will usually be a dye or fluorescer. A large number of illustrative fluorescers are indicated in U.S. Patent No. 4,275,149, columns 30 and 31.

The signal producing system can include one or more particles, which are insoluble particles of at least about 50 nm and not more than about 50 microns, usually at least about 100 nm and less than about 25 microns, preferably from about 0.2 to 5 microns, diameter. The particle may be organic or inorganic, porous or non-porous, preferably of a density approximating water, generally from about 0.7 to about 1.5 g/ml, and composed of material that can be transparent, partially transparent, or opaque.

The organic particles will normally be comprised of polymers, either addition or condensation polymers, which are readily dispersible in the assay medium. The surface of particles will be adsorptive or functionalizable so as to bind, either directly or indirectly, an oligonucleotide or an sbp member. The nature of particles is described above.

Fluorescers of interest will generally emit light at a wavelength above 350nm, usually above 400nm and preferably above 450nm. Desirably, the fluorescers have a high quantum efficiency, a large Stokes shift and are chemically stable under the conditions of their conjugation and use. The term fluorescer is intended to include substances that emit light upon activation by electromagnetic radiation or chemical activation and includes fluorescent and phosphorescent substances, scintillators, and chemiluminescent substances.

Fluorescers of interest fall into a variety of categories having certain primary functionalities. These primary functionalities include 1- and 2-aminonaphthalene, p,p-diaminostilbenes, pyrenes, quaternary phenanthridine salts, 9-aminoacridines, p,p′-diaminostilbenes immines, anthracenes, oxacarboxyanine, merocyanine, 3-aminoequilenin, perylene, bis-benzoxazole, bis-p-oxazolyl benzene, 1,2-benzophenazine, retinal, bis-3-aminopyridinium salts, hellebrigenin, tetracycline, sterophenol, benzimidazolylphenylamine, 2-oxo-3-chromen, indole, xanthene, 7-hydroxycoumarin, 4,5-benzimidazoles, phenoxazine, salicylate, strophanthidin, porphyrins, triarylmethanes, flavin and rare earth chelates oxides and salts. Exemplary fluorescers are enumerated in U.S. Patent No. 4,318,707, columns 7 and 8.

Additionally, energy absorbent or quenching particles can be employed which are solid insoluble particles of at least about 50 nm in diameter capable of quenching the fluorescence of the fluorescent particle when within the distance resulting from hybridization of a probe with the polynucleotide analyte or from specific binding between members of specific binding pairs. The quenching particle may be the same or different, usually different, from the fluorescent particle. Normally, the quenching particle will provide a substantial quenching at a distance of more than about 50Å, preferably more than about 500Å, more preferably more than about 2000Å, where the distance is measured from the surfaces of the particles.

Many different types of particles may be employed for modulating light emission. Of particular interest are carbon particles, such as charcoal, lamp black, graphite, colloidal carbon and the like. Besides carbon particles metal sols may also find use, particularly of the noble metals, gold, silver, and platinum. Other metal-derived particles may include metal sulfides, such as lead, silver or copper sulfides or metal oxides, such as iron or copper oxide.

An alternative source of light as a detectible signal is a chemiluminescent source. The chemiluminescent source involves a compound which becomes electronically excited by a chemical reaction and may then emit light which serves as the detectible signal or donates energy to a fluorescent acceptor.

A diverse number of families of compounds have been found to provide chemiluminescence under a variety of conditions. One family of compounds is 2,3-dihydro-1,4-phthalazinedione. The most popular compound is luminol, which is the 5-amino analog of the above compound. Other members of the family include the 5-amino-6,7,8-trimethoxy- and the dimethylamine-[ca]benz analog. These compounds can be made to luminesce with alkaline hydrogen peroxide or calcium hypochlorite and base. Another family of compounds is the 2,4,5-triphenylimidazoles, with lophine as the common name for the parent product. Chemiluminescent analogs include para-dimethylamino- and para-methoxy-substituents. Chemiluminescence may also be obtained with oxilates, usually oxalyl, active esters, e.g., p-nitrophenyl and a peroxide, e.g., hydrogen peroxide, under basic conditions. Alternatively, luciferins may be used in conjunction with luciferase or lucigenins.

"Ancillary materials" means various additional materials employed in an assay in accordance with the present invention. For example, buffers will normally be present in the assay medium, as well as stabilizers for the assay medium and the assay components. Frequently, in addition to these additives, additional proteins may be included, such as albumins, or surfactants, particularly non-ionic surfactants, binding enhancers, e.g., polyalkylene glycols, or the like.

"Wholly or partially sequentially" means when the sample and various agents utilized in the present invention are combined other than concomitantly (simultaneously), one or more may be combined with one or more of the remaining agents to form a subcombination. Each subcombination can then be combined and subjected to the present method.

As mentioned above, one aspect of the present invention involves a method for carrying out an immunoassay for a specific immunoglobulin wherein the following components are combined in an aqueous medium: a sample suspected of containing the immunoglobulin, a small molecule bound to a first antigen capable of binding to the immunoglobulin, signal generating means bound to a second antigen capable of binding to the immunoglobulin, and a support to which is bound a receptor for the small molecule. The first and second antigens can be the same antigen, the only difference being that some antigen will be bound to the small molecule and some antigen will be bound to the signal generating means. The medium is then separated from the support. The medium or the support is observed for the presence or amount of the signal generating means, the presence or amount thereof being related to the presence or amount of immunoglobulin in the sample. In a preferred practice of the invention, the solid support is a matrix of beads.

The reagents used in the immunoassay of this invention are of particular importance in the design of this invention. As designed, the methodology consists of certain reagents which are generic to most analytes, thereby providing for easier automation and control of stability of reagents in an immunoassay systems. Typically, the present invention uses the following reagents in an assay for an immunoglobulin of interest: (1) a small molecule bound to a first antigen capable of binding to the immunoglobulin, (2) a signal generating means bound to a second antigen capable of binding to the immunoglobulin, and (3) a support to which is bound a receptor for the small molecule. If the same small molecule is used in all assays, then the support bound to a receptor is a generic reagent as it can be used regardless of what the analyte is. The signal generating means can consist of a second small molecule bound to a receptor for the second antigen along with a labeled receptor for the second small molecule. Once again, if the same second small molecule is used in all assays, then the labeled receptor for the second small molecule is a generic reagent.

In one practice of the invention, a methodology for the detection of an immunoglobulin in a sample is set forth. The methodology of this assay utilizes the following reagents: a sample suspected of containing an immunoglobulin (Ig) of interest; a small molecule bound to a first antigen capable of binding to Ig, for example, biotin bound to an antigen; a signal generating means bound to a second antigen capable of binding to Ig, for example, a label bound to an antigen; and a support to which is bound a receptor for the small molecule, for example, avidin. In another embodiment, the signal generating means can be a label bound to a receptor for a small molecule, where a small molecule is bound to the second antigen, for example, enzyme labeled anti-fluorescein and fluorescein labeled antigen.

The amount of signal generating means that will become bound to the solid support will be directly related to the amount of immunoglobulin present in the sample. In particular, in the presence of Ig, antigen bound to the signal generating means will be bound to Ig. In turn, the antigen to which is bound a small molecule, binds to Ig and the small molecule binds to the receptor for the small molecule, which is bound to the support. This results in the complex of interest binding to the support.

The reagents and sample can be combined simultaneously or wholly or partially sequentially with each other and with the solid support. In one approach, all reagents, the sample and the solid support are mixed together. This is followed by a single incubation, after which unbound reagents are removed by washing. A schematic representation of the complex formed in the immunoassay of the invention as applied to detection of IgG-type immunoglobulins is as follows:

$$\text{Label-Ag}_2\text{: IgG: Ag}_1\text{-X:Y-support}$$

where $\text{Ag}_1\text{-X}$ is a small molecule (X) bound to a first antigen ($\text{Ag}_1$) capable of binding to IgG; Label-$\text{Ag}_2$ is a label bound to a second antigen ($\text{Ag}_2$) capable of binding to IgG; and Y-support is a receptor for the small molecule bound to a support. In a preferred embodiment, the first and second antigens are hepatitis B surface antigens. X can be biotin and Y can be avidin.

In one embodiment of the invention, the label, an enzyme for example, can be bound directly to the antigen. The degree of enzymatic activity then correlates to the concentration of IgG in the sample. In a variation on such an assay, a small molecule such as fluorescein, is bound to the antigen. The label is then bound to an anti-fluorescein antibody.

The design of the assay system makes it possible to use reagents which are generic, i.e. can be used in any assay system no matter what the analyte of interest. As is apparent in the above examples, each assay uses a receptor for a small molecule bound to the support.

In carrying out the assay there are certain sequences of steps that are more convenient and the choice of the particular sequence to be employed will therefore depend upon the needs of the person performing the assay. In general, the sample, all of the reagents and the support are combined in an aqueous medium, usually without regard to the order of addition. Typically, the sample, reagents and support are combined essentially simultaneously. The aqueous medium containing the sample, reagents and support, is then incubated for a period of up to an hour or more. The amount of signal generating means bound to the support or remaining in the medium may then be measured directly but will usually be measured following separation of the medium from the support.

In the assay of the present invention, any convenient label may be used. The label will normally be bonded covalently to an antigen. However, this invention also contemplates having a small molecule bound to the antigen and having the label bound covalently to a receptor for the small molecule, usually an antibody. Bonding may be accomplished by chemical reactions wherein the result is replacing a hydrogen atom of the label with a bond to the antigen or may include a linking group between the label and the antigen. The linking group may be of any size, but preferably no larger than necessary to permit unfettered binding of the immunoglobulin to the antigen bound to the label and to permit signal production by the label. Generally, the linking group will be a bond or a group of from 1 to 100 atoms, usually from 1 to 15 atoms. The linking group can be introduced into the label or the receptor for attachment. A functionality for attachment such as carboxylic acid, hydroxyl, thio, amino, aldehydic, amido, activated ethylenese such as maleimide, sulfonic acids, and the like can be introduced into the label or the receptor if the functionality is not originally present in the label or receptor. Methods of conjugation are well known in the art. See for example, U.S. Patent No. 3,817,837.

Conjugates of this invention in which an antigen is bound to a small molecule, will contain small molecules for which a natural receptor exists or can be prepared. The small molecules will usually be neither extremely hydrophilic nor extremely hydrophobic and will preferably be structurally dissimilar to substances that are likely to be present in the sample. Conjugates of small molecules with antigens that have multiple determinant sites will have at least one and frequently 2-20 small molecules in the conjugate which will usually be bound covalently. As described above for the label-antigen chemical reaction, bonding of the small molecule to the antigen may be accomplished by chemical reactions that result in replacing a hydrogen atom of the small molecule with a bond to the antigen or may include a linking group of any size but preferably no larger than necessary to permit binding to the conjugate of both a receptor for the small molecule and the immunoglobulin to the antigen in the conjugate.

The support in this invention will normally be particulate such as beads, liposomes, cells, sols, and the like; bibulous materials such as porous membranes, cellulosic paper, glass paper, and nitrocellulose membranes; or non-porous materials such as glass, plastic, metal, ceramic, and the like. The receptor can be directly bonded, either covalently or non-covalently, to the surface of the support in such a manner as to permit ready binding of the conjugate containing a small molecule complementary to the receptor. Bonding of the receptor to the surface will normally be achieved by incubating the surface with the receptor, wherein the surface may previously have been treated with a reagent to enhance binding such as polycations, for example polylysine; carbodiimides; silylating agents; bifunctional cross linking reagents such as carbonyl diimidazole; periodate; and similar activating reagents. Alternatively, the receptor can be indirectly bound to the support by first preparing the support with a compound complementary to the receptor such as the ligand for the receptor or an antibody against the receptor already bound to the surface. Incubation of the receptor with such a surface will then cause the receptor to bind non-covalently. When this method of binding is employed wherein a ligand for the receptor is initially present on the surface, the receptor will normally have at least two binding sites for the ligand.

In the invention described herein, when the solid support is a matrix of beads, the beads are usually nonporous, usually glass or latex and normally are 0.2-2.5 mm average in diameter. Most preferably, the beads are 0.5-2 mm average in diameter. The beads are usually approximately spherical and may have a rough or smooth surface.

Because of the high surface area of beads, attention must be paid to the surface properties so that background nonspecific binding remains low. Where avidin is used as the receptor bound to the beads, non-specific binding can be reduced by drying the glass particles in the presence of sucrose after the binding of avidin to the beads. Examples of coatings in addition to sugars, which have been found useful include bovine serum albumin (BSA), poly(vinylalcohol), casein and non-fat milk.

Whatever type of solid support is used it must be treated so as to have a receptor bound to its surface, which receptor will specifically bind to a small molecule. In a preferred practice of the invention, the support will have bound to it a ligand or receptor that will permit the support to be used for a variety of different assays. For example, avidin can be covalently bound to spherical glass beads of 0.5-1.5 mm. A matrix of these beads is mixed in an aqueous medium with biotin-labeled antigen capable of binding to the immunoglobulin, a sample containing the immunoglobulin, and a labeled antigen that is capable of binding to the immunoglobulin. Because the beads bind to biotin and biotin can be bound to any antigen, the same beads can be used for most antigen-immoglogulin pairs. After sufficient incubation to permit binding of the labeled antigen, the immunoglobulin, the biotinylated antigen and binding of the latter to the beads, the solution is separated from the beads, for example, by aspiration. Wash solution is then added and liquid is again removed. After repeating the wash cycle, the label is detected and the amount of label is related to the amount of Ig in the sample. For example, if the label is an enzyme, substrate would be added and the amount of enzyme product determined photometrically after a suitable incubation time and compared to the amount of product provided using a sample of known concentration of Ig.

In carrying out the invention, a liquid, usually aqueous, medium will be employed. Other polar solvents may also be employed, usually oxygenated organic solvents from one to six, more usually from one to four, carbon atoms, including alcohols, ethers, and the like. Usually these cosolvents will be present in less than about 40 weight percent, more usually in less than about 20 weight percent. Generally, a pH range of 5 to 10, more usually 6 to 9, will be used. One consideration with respect to the pH of the assay is the maintenance of a significant level of binding while optimizing signal producing proficiency. In some instances, a compromise will be made between these considerations. Various buffers may be used to achieve the desired pH and maintain the pH during the determination. Illustrative buffers include borate, phosphate, carbonate, Tris, barbital, and the like. The particular buffer employed is not critical to this invention; however, in individual separations or individual assays, one buffer may be preferred over another.

Moderate, usually constant, temperatures are normally employed for carrying out the assay. The temperature for the assay, particularly involving an immunoassay, will generally range from about 0-50°C, more usually from about 15-40°C.

While the concentrations of the various reagents will generally be determined by the concentration range of the antigens in the liquid medium or of the immunoglobulin in an assay, the final concentration of each of the reagents will normally be determined empirically to optimize the sensitivity and specificity of the separation or of the assay over the range of interest.

In the immunoassay of the invention, the aqueous medium can also contain one or more members of a signal generating means. The concentration of the various members of the signal generating means will vary and be dependent upon the concentration range of interest of the immunoglobulin and the type of measurement or assay involved. As a general point, the concentration of the various members of the signal generating means will be selected to optimize the sensitivity of the assay within the concentration range of interest of the immunoglobulin.

In order to determine the results of the assay, the labeled solid phase reaction product, such as:

Label-$Ag_2$: IgG: $Ag_1$-X:Y-support

is preferably separated from the aqueous medium, which may contain free reagents which were present in an excess amount and therefore are unreacted. Since the reaction product is a solid phase insolubilized by the support, it can be readily separated by methods such as sedimentation or centrifugation, or other methods well established in the art.

As a matter of convenience, the reagents for conducting an assay can be provided in a kit in packaged combination in predetermined amounts for use in assaying for an immunoglobulin (Ig). The kit comprises (a) a conjugate of a small molecule and a first antigen capable of binding to Ig, (b) a conjugate of a label and a second antigen capable of being bound to Ig, and (c) a support comprised of a surface bound to a receptor for the small molecule. The kit can also include other reagents for generating a signal in relation to the amount of Ig in the sample, for example, a substrate for the label. Ancillary agents can be included as necessary.

Use of the method of the invention is applicable to any heterogeneous binding assay for an immunoglobulin. Specific assays include for example, an assay for IgG antibodies for hepatitis B surface antigen. In any such system, biotinylated antigen that is capable of binding to the immunoglobulin is used. Receptors other than avidin (which includes streptavidin) may be attached to the beads, such as antibodies, protein A, intrinsic factor, Protein G, C1q, lectins, apoenzymes and the like, whereupon the respective complementary small molecule conjugated to the antigen is then used.

Certain preferred embodiments of the present invention have the following significant advantages over the standard ELISA chemistry: 1) receptor bound supports are generic to all assays; 2) the binding of antigen to immunoglobulin in solution phase results in very rapid kinetics compared to those achieved with antigen immobilized on a solid surface; 3) elimination of the need for separate incubations and separate washings provides an efficient assay with a minimum number of manipulative steps. The chemistry to link small molecules such as biotin or fluorescein to antigens or receptors is simple and efficient. (See, for example, D.M. Boorsma, Immunocytochemistry 2:155 (1983)). The stability of the small molecule conjugates will be as good as the antibodies used in the conjugate.

## Examples

The examples which follow are illustrative and not limiting of the invention. Unless otherwise indicated, reagents were obtained from commercial sources and, where applicable, were used according to manufacturer's directions.

The following abbreviations are used throughout the examples:

| | |
|---|---|
| anti-F | anti-fluorescein antibody |
| anti-HBsAg | anti-hepatitis B surface antigen antibody |
| BSA | bovine serum albumin |
| DMF | dimethyl formamide |
| DMSO | dimethyl sulfoxide |
| EDAC | 1-ethyl-(3-dimethylaminopropyl) carbodiimide |
| EDTA | ethylenediaminetetraacetic acid, tetrasodium salt |
| F | fluorescein |
| GB | glass beads |
| HBsAg | hepatitis B surface antigen |
| IgG | immunoglobulin G |
| HRP | horseradish peroxidase |
| LC | 3,3′-diamino-N-methyldipropylamine |
| MES | 2-[N-morpholino]ethane sulfonic acid |
| NHS | N-hydroxysuccinimide |
| NHS-LC-biotin | succinimidyl 6-(biotinamido) hexanoate |
| PBS | phosphate-buffered saline |
| SAMSA | S-acetylmercaptosuccinic acid |
| SMCC | succinimidyl 4-(N-maleimidomethyl cyclohexane 1-carboxylate |
| TMB | 3,3′,5,5′-tetramethylbenzidine-2 HCl |

EXAMPLE 1

Heterogeneous enzyme-based immunoassay for detection of immunoglobulin G for hepatitis B surface antigen

Preparation of Materials

A. Preparation of Biotin-HBsAg

The starting material, HBsAg was purchased 98% pure from Scantibodies Lab. Inc. (Santee, CA). Before the reaction, HBsAg at 1.0 mg/ml was dialyzed against pH 8.2 NaHCO$_3$ buffer at 4°C overnight. NHS-LC-biotin was solubilized in DMSO at 15 mM and added into HBsAg (around 1.0 mg/ml) drop-wise while stirring. This mixture was then incubated at room temperature for 1.5 hours. Uncoupled biotinylated reagent was removed by SEPHADEX® G-25 column (beaded gel prepared by cross-linking dextran with epichlorohydrin under alkaline conditions) or dialysis against pH 7.5 PBS. The molar ratio of HBsAg to NHS-LC-biotin was 1:5. The molecular weight of HBsAg used for the calculation was 25,000 daltons which is the molecular weight of one of the major polypeptides of the antigen. The conjugate was stored at 4°C.

B. Preparation of HBsAg-F

HBsAg-F was prepared using SMCC/SAMSA chemistry. First, carboxyfluorescein was activated using NHS/EDAC chemistry and then coupled to oxybis(ethylamine). The resulting fluoresceinated amine was then treated with SMCC to yield F-SMCC.

HBsAg at 1.0 mg/ml in 100 mM carbonate buffer at pH 9.5 was reacted with 12 times molar excess of SAMSA which was dissolved in DMF at 100 mM. The resulting derivatized HBsAg was then deacylated to 500 mM NH$_2$OH to yield HBsAg-SH. This reaction was carried out for one hour in a reacti-vial which was filled with argon. Excess NH$_2$OH was removed by dialysis against pH 7.0 PBS.

HBsAg-SH and F-SMCC were mixed at the molar ratio of 1:10 and reacted at room temperature for one hour. After the reaction, the mixture was run though a SEPHADEX G-25 column or dialyzed against PBS at pH 7.5. The prepared conjugate was stored in the dark at 4°C.

C. Preparation of anti-F-HRP

Anti-F-HRP conjugate was prepared by reacting thiolated anti-F with maleimidated HRP, using sulfo-SMCC/SAMSA chemistry. Before the conjugation, anti-F which was purified using a Protein A column, was thiolated with SAMSA. HRP was maleimidated with sulfo-SMCC.

The-HRP at 9.6 mg/ml in 50 mM borate buffer, pH 7.6, was reacted with 10 fold excess of sulfo-SMCC. The reaction was allowed to proceed for 2 hours at 25°C. After the reaction, the mixture was passed through a SEPHADEX G-25 column and dialyzed against a phosphate buffer (100 mM NaH$_2$PO$_4$ and 100 mM NaCl, pH 7.5) containing 5 mM EDTA.

Anti-F at 17.9 mg/ml in 50 mM bicarbonate buffer, pH 9.5, was reacted with 24 fold excess of SAMSA which was dissolved in 100 mM DMF. The addition of SAMSA was drop-wise while stirring and reacted at 4°C for 1.5 hours. Excess reagent was removed by a SEPHADEX G-25 column and dialysis against a phosphate buffer at pH 7.5.

Derivatized anti-F was then deacylated by treating with 1 M NH$_2$OH. NH$_2$OH in a phosphate buffer was slowly added while stirring until the final concentration of NH$_2$OH reached 100 mM. The reaction mixture was incubated under nitrogen for 1 hour at 25°C. Excess NH$_2$OH was removed by a SEPHADEX G-25 column.

Finally, the resulting anti-F-SH was reacted with 12 fold excess of maleimidated HRP and stirred for 3 hours at room temperature. The reaction was stopped by adding 2-mercaptoacetic acid (at a final concentration of 1 mM) and stirred for 15 minutes. Iodoacetic acid was added (at a final concentration of 2 mM) to quench the excess of 2-mercaptoacetic acid and reacted for 5 minutes. The final product was purified by SEPHACRYL® S-200 column. The conjugate concentration was 1.4 mg/ml with 6.2 HRP per IgG.

D. Preparation of GB-Avidin

Glass beads of approximately 1 mm in diameter (Glen Mills, Inc., Maywood, NJ) were first cleaned by boiling in 5% nitric acid for one hour and then washed with deionized water until the wash was neutral in pH. The beads were dried at room temperature under vacuum.

To 1 kg of the acid-washed beads was added 1 mL of aminopropyltriethoxysilane in 300 mL ethyl acetate. The mixture was then placed on a rotary aspirator, and upon removal of the solvent, the beads were coated with a thin film of the aminosilane reagent. The beads were then transferred to a stainless reactor and heated in an oven at 130°C overnight under nitrogen/argon atmosphere. After cooling, the beads were used directly in the next step.

To 500 g of the aminated beads in a canted tissue culture flask was added 170 mL 0.1 M of sodium borate pH 9.0 for 10 minutes. A solution of succinic anhydride mg) of EDAC in 2 mL MES was added in one portion and mixed for 5 minutes with manual shaking. Upon removal of the liquid by use of an aspirator, a 20 mL MES solution of avidin (20 mg) and BSA (40 mg) was added in one portion. The beads were mixed manually and more MES buffer was added to just cover the beads. Finally, the culture flask with its contents was placed on an orbital shaker overnight at 4°C.

Further preparation of the beads included washing the beads with 1 N NaCl (200 mL x 4) followed by deionized water (200 mL x 4). Before and after each wash, the liquid in removed entirely. The beads are then treated with a phosphate-saline buffer (20 mM phosphate, 140 mM NaCl 0.02% $NaN_3$, pH 7.4) containing 0.1% BSA and 2.5% sucrose (150 mL x 3). Excess liquid is removed and the wet beads are transferred to a container in a vacuum dessicator.

Finally, after passage through a number 16 or 20 USA Standard Testing Sieve, the beads were dusted with casein powder to prevent sticking together upon storage.

Binding study with $^3$H-biotin indicated that the beads thus prepared incorporated 2-11 mg active avidin-/glass beads.

Assay Protocol

The one step anti-HBsAg assay consisted of two main parts: 1) the simultaneous incubation of all the assay components (GB-avidin, biotin-HBsAg, anti-HBsAg from the sample, HBsAg-F and anti-F-HRP); and 2) the addition of substrate for generating color. All reactions took place in 12 x 75 mm test tubes.

First, 100 ml of unknown serum samples (or standard), 50 ml of biotin-HBsAg (50 ng/test), and 50 ml of enzyme conjugate mixture (containing 200 ng of F-HBsAg and 100 ng of anti-F-HRP) were added to a test tube containing 0.65 g of GB-avidin. This reaction mixture was incubated at 37°C for 30 minutes.

Second, unbound reagents were washed away four times with 1 ml of 10 mM phosphate buffer at pH 7.4. After the wash, 250 ml of HRP substrate (TMB/urea $H_2O_2$) was added to generate color at 37°C for 5 minutes.

Finally, the substrate developing reaction was stopped by the addition of 750 ml of 1 N $H_2SO_4$. The optical density was read at 450 nM.

Results

Positive (800 mIU/ml) and negative anti-HBsAg serum control were run by the one-step protocol. The results were as follows:

| Control | Signal at OD 450 nM |
|---------|---------------------|
| Positive | 2.31 |
| Negative | 1.03 |

Therefore, specific to non-specific signal ratio was 1.2 (1.28/1.03). Further, protocol optimization, which includes conjugate loading and replacing conjugates HBsAg-F and anti-F-HRP with the direct conjugate HBsAg-HRP, may improve the assay performance by reducing the non-specific signal.

The above discussion includes certain theories as to mechanisms involved in the present invention. These theories should not be construed to limit the present invention in any way, since it has been demonstrated that the present invention achieves the results described.

The invention has been described in detail with particular reference to the above embodiments. It will be understood, however, that variations and modifications can be effected within the spirit and scope of the invention.

## Claims

1. A method for carrying out an immunoassay for a specific immunoglobulin comprising the steps of:
   (a) providing in combination in an aqueous medium
      (i) a sample suspected of containing immunoglobulin,
      (ii) a small molecule bound to a first antigen capable of binding to said immunoglobulin, (iii) a signal generating means bound to a second antigen capable of binding to said immunoglobulin, and (iv) a support to which is bound a receptor for said small molecule;
   (b) incubating said combination;
   (c) separating said medium and said support; and
   (d) observing said medium or said support for the presence or amount of a signal, the presence or amount thereof being related to the presence or amount of said immunoglobulin in said sample.

2. The method of claim 1 wherein said immunoglobulin is directed against a causative agent of a disease state.

3. The method of claim 1 or claim 2 wherein said immunoglobulin is an IgG antibody.

4. The method of any one of the preceding claims wherein said first and second antigens are hepatitis B surface antigens.

5. The method of any one of the preceding claims wherein said signal generating means is a label selected from the group consisting of enzymes, fluorophors, chemiluminescers and metallic particles, preferably wherein said label is an enzyme selected from the group consisting of peroxidase, β-galactosidase, urease, alkaline phosphatase, and Q-beta-replicase.

6. The method of any one of claims 1 to 4 wherein said signal generating means comprises a second small molecule bound to said second antigen and a receptor for said second small molecule bound to a label selected from the group consisting of enzymes, fluorophors, chemiluminescers and metallic particles, preferably wherein said molecule is a fluorescein derivative, said receptor is an antibody and said label is an enzyme selected from the group consisting of peroxidase, β-galactosidase, urease, alkaline phosphatase, and Q-beta-replicase.

7. The method of any one of the preceding claims wherein said small molecule is biotin, and wherein said receptor for said small molecule is selected from the group consisting of avidin and streptavidin.

8. The method of any one of the preceding claims wherein said support is glass beads.

9. A method for carrying out an immunoassay for an IgG-type immunoglobulin comprising the steps of:
   (a) providing in an assay medium (i) a sample suspected of containing IgG, (ii) a biotinylated first antigen capable of binding to said IgG, (iii) a signal generating means bound to a second antigen capable of binding to said IgG and (iv) a receptor for biotin bound to a support;
   (b) incubating said medium;
   (c) separating said medium and said support; and
   (d) observing said medium or support for the presence or amount of a signal, the presence or amount thereof being related to the presence or amount of IgG in said sample.

10. An assay for detecting an IgG-type immunoglobulin suspected of being present in a sample comprising the step of forming in direct relation to the amount of said IgG in said sample a complex
    $$\text{Label-Ag}_2: \text{IgG}: \text{Ag}_1\text{-X:Y-support}$$
    where $\text{Ag}_1\text{-X}$ is a small molecule (X) bound to a first hepatitis B surface antigen ($\text{Ag}_1$) capable of being bound to said IgG; $\text{Label-Ag}_2$ is a label bound to a second hepatitis B surface antigen ($\text{Ag}_2$) capable of being bound to said IgG; and Y-support is a receptor to said small molecule bound to a support.

11. A composition of matter consisting of a conjugate of a biotinylated first antigen bound to an immunoglobulin and a labeled second antigen bound to said immunoglobulin.

12. The composition of claim 11 wherein said biotinylated antigen is further bound to a support having a receptor for biotin, said receptor being selected from the group consisting of avidin and streptavidin.

13. A composition of matter:

$$\text{Label-}Ag_2 : \text{IgG} : Ag_1\text{-X:Y-support}$$

wherein IgG is an IgG-type immunoglobulin; $Ag_1$-X is a small molecule (X) bound to a first hepatitis B surface antigen ($Ag_1$) capable of binding to IgG; Label-$Ag_2$ is a label bound to a second hepatitis B surface antigen ($Ag_2$) capable of binding to IgG; and Y-support is a receptor to said small molecule bound to a support.

14. A kit for carrying out an immunoassay for an immunoglobulin comprising in packaged form: (a) a conjugate of a small molecule and a first antigen capable of binding to said immunoglobulin; (b) a conjugate of a label and a second antigen capable of being bound to said immunoglobulin; and (c) a support comprised of a surface bound to a receptor for said small molecule.